Europäisches Patentamt

European Patent Office

Office européen des brevets

(19)

(11) EP 0 625 137 B1

(12) **EUROPÄISCHE PATENTSCHRIFT**

(45) Veröffentlichungstag und Bekanntmachung des Hinweises auf die Patenterteilung:
**09.04.1997 Patentblatt 1997/15**

(21) Anmeldenummer: **93903886.5**

(22) Anmeldetag: **28.01.1993**

(51) Int Cl.6: **C07C 255/26**

(86) Internationale Anmeldenummer:
**PCT/EP93/00196**

(87) Internationale Veröffentlichungsnummer:
**WO 93/16035 (19.08.1993 Gazette 1993/20)**

(54) **VERFAHREN ZUR RACEMATTRENNUNG VON VERAPAMIL**

METHOD OF SEPARATING RACEMATES OF VERAPAMIL

PROCEDE DE SEPARATION DES RACEMATES DU VERAPAMIL

(84) Benannte Vertragsstaaten:
**AT BE CH DE DK ES FR GB IT LI NL SE**

(30) Priorität: **07.02.1992 DE 4203547**

(43) Veröffentlichungstag der Anmeldung:
**23.11.1994 Patentblatt 1994/47**

(73) Patentinhaber: **KNOLL AKTIENGESELLSCHAFT**
**D-67061 Ludwigshafen (DE)**

(72) Erfinder:
 • **EHRMANN, Oskar**
 **D-6800 Mannheim-Neuhermsheim (DE)**
 • **NAGEL, Herbert**
 **D-6707 Schifferstadt (DE)**

(74) Vertreter: **Karau, Wolfgang, Dr.**
**BASF Aktiengesellschaft,**
**Patentabteilung ZDX - C 6**
**67056 Ludwigshafen (DE)**

(56) Entgegenhaltungen:
 **EP-A- 0 029 175     DE-A- 3 723 684**

 • **HELVETICA CHIMICA ACTA. Bd. 58, Nr. 7, 1975, BASEL CH Seiten 2050 - 2060 H. RAMUZ 'SYNTHESE ET CONFIGURATION ABSLUE DES ENATIOMERES DU VERAPAMIL' in der Anmeldung erwähnt**

## Beschreibung

Verapamil [1,7-Bis-(3,4-dimethoxyphenyl)-3-methylaza-7-cyan-8-methylnonan] ist bereits aus der DE-PS 1 154 810 bekannt. Diese racemischen Verbindung konnte lange Zeit nicht in die optischen Antipoden getrennt werden (Helv. Chim. Acta 58, 2050 (1975)). Man war deshalb auf deren Synthese aus optisch aktiven Vorstufen angewiesen (DE-PS 20 59 985, DE-PS 20 59 923).

In der EP-PS 29 175 ist die Racematspaltung von Gallopamil mit Hilfe des O,O'-Dibenzoylderivates der unnatürlichen (+)-D-Weinsäure beschrieben. Die Racematspaltung von Verapamil wird in der EP-PS jedoch nicht beschrieben.

Hingegen wird in der DE-OS 3 723 684 ein Verfahren zur Racemattrennung von Verapamil mit Hilfe von R- oder S-2,2'-(1,1'-Binaphthyl)-phosphorsäure beschrieben. Diese Substanz ist jedoch äußerst teuer in ihrer Herstellung, so daß eine wirtschaftliche Herstellung der Antipoden des Verapamils nach wie vor nicht möglich ist.

Nach diesem Stand der Technik mußte man davon ausgehen, daß eine Racematspaltung von Verapamil sehr schwierig und aufwendig ist.

Es wurde nun ein sehr günstiges Verfahren gefunden, mit dessen Hilfe man Verapamil in seine Antipoden spalten kann.

Gegenstand der Erfindung ist ein Verfahren zur Racemattrennung von Verapamil, dadurch gekennzeichnet, daß man die freie Base der Verbindung mit optisch aktiver Dibenzoylweinsäure oder Ditoluoylweinsäure im Molverhältnis 1:1 bis 1:2 in einem Methanol-Wasser-Gemisch im Verhältnis 1:1 bis 3:1 oder Aceton-Wasser-Gemisch im Verhältnis 0,5:1 bis 2:1 umsetzt, das so erhaltene Diastereomerengemisch durch Kristallisation trennt und die so erhaltenen Diastereomeren in die freien Basen und anschließend gegebenenfalls in ihre Salze überführt.

Verapamil und die optisch aktive Säure werden im Molverhältnis 1:1 bis 1:2, vorzugsweise 1:1,5 miteinander umgesetzt. Aus der Lösung in Methanol/Wasser bzw. Aceton/Wasser kristallisiert beim Abkühlen das Salz eines Antipoden aus, während der andere Antipode in der Mutterlauge verbleibt. Verwendet man als Säure (-)-O,O'-Dibenzoyl-L-weinsäure, so kristallisiert zuerst das Dibenzoyl-L-tartrat des (S)-Verapamils aus, verwendet man (+)-O,O'-Dibenzoyl-D-weinsäure, so kristallisiert zuerst das Dibenzoyl-D-tartrat des (R)-Verapamils aus.

Nach Abtrennen des ausgefallenen diastereomeren Salzes wird das Lösungsmittel abdestilliert, aus dem Rückstand die Base freigesetzt und diese mit der anderen enantiomeren Form der O,O'-Dibenzoylweinsäure in das Hydrogentartrat übergeführt. Entsprechendes gilt für die Ditoluylweinsäuresalze. Vorteilhafterweise werden für die Racemattrennung die Dibenzoyl- bzw. Ditoluoylderivate der natürlichen L-Weinsäure eingesetzt.

Besonders vorteilhaft und kostengünstig läßt sich die Racematspaltung des Verapamils durch Zusatz einer anorganischen Mineralsäure gestalten. Dadurch lassen sich bis zu 1,5 Mol der Weinsäure pro Mol Verapamil einsparen. Am günstigsten ist es, die Spaltung mit optisch aktiver Dibenzoyl-oder Ditoluoylweinsäure in Gegenwart von Mineralsäure im Molverhältnis von 1 : 0,75 : 0,5 (Verapamil : Weinsäure : Mineralsäure) durchzuführen. Das kristallisierende diastereomere Salz des einen Enantiomeren ist von gleicher optischer Reinheit. Das andere Enantiomere bleibt in Lösung und kann daraus in üblicher Weise, beispielsweise durch Einengen der Lösung, isoliert werden. Als Mineralsäuren eignen sich vor allem Phosphorsäure und Schwefelsäure, bevorzugt wird mit Salzsäure gearbeitet.

Aus den diastereomeren Salzen wird das (R)- bzw. (S)-Verapamil in üblicher Weise mit Hilfe einer Base in wäßrigem Medium freigesetzt und durch Extraktion isoliert. Die so erhaltenen Basen können nach bekannten Verfahren in ihre Salze mit physiologisch verträglichen Säuren überführt werden.

Beispiel 1

a) 250 g (0,55 Mol) racemisches Verapamil und 310 g (0,82 Mol) (-)-O,O'-Dibenzoyl-L-weinsäure-hydrat wurden unter Erwärmen in 1,3 l Methanol/Wasser (2:1) gelöst. Das über Nacht ausgefallene Kristallisat wurde abgesaugt und getrocknet. Die Kristalle haben einen Schmelzpunkt von 103 bis 107°C und einen Drehwert $[\alpha]_D^{20} = -53,3°$ (Ethanol, c = 15 mg/ml). Man kristallisierte dreimal aus Methanol/Wasser = 2:1 um und erhielt ein Kristallisat mit dem Schmelzpunkt 113 bis 115°C sowie dem Drehwert $[\alpha]_D^{20} = -66,2°$ (Ethanol, c = 15 mg/ml). Dieser Wert veränderte sich bei nochmaliger Umkristallisation nicht mehr. Die aus dem Salz mit Natronlauge freigesetzte Base wurde direkt in das Hydrochlorid überführt. Nach einmaligem Umkristallisieren aus Diisopropylether/Isopropanol (3:2) erhielt man 83,7 g (62 %) (S)-Verapamilhydrochlorid mit dem Schmelzpunkt 131 bis 133°C und dem Drehwert $[\alpha]_D^{20} = -9,2°$ (Ethanol, c = 50 mg/ml). Der Wert änderte sich durch nochmalige Kristallisation nicht mehr.

b) Die in a) bei der Ausfällung angefallene Mutterlauge wurde im Vakuum eingeengt, der Rückstand in Wasser aufgenommen und daraus durch Zugabe von 1 M Natronlauge die Base freigesetzt. Nach Extraktion mit Toluol, Trocknen über Natriumsulfat und Abdestillieren des Lösungsmittels wurde ein Öl isoliert.

Dieses öl und 155 g (0,41 Mol) (+)-O,O'-Dibenzoyl-O-weinsäurehydrat wurden unter Erwärmen in 650 ml Methanol/Wasser = 2:1 gelöst. Das über Nacht ausgefallene Kristallisat wurde abgesaugt und getrocknet. Die Kristalle haben einen Schmelzpunkt von 105 bis 109°C und einen Drehwert von $[\alpha]_D^{20} = +61{,}5°$ (Ethanol, c = 15 mg/ml). Das Salz wurde zweimal aus Methanol/Wasser 2:1 umkristallisiert. Man erhielt ein Kristallisat vom Schmelzpunkt 113 bis 115°C und einem Drehwert von $[\alpha]_D^{20} = +66{,}8°$ (Ethanol, c = 15 mg/ml). Der Wert änderte sich durch nochmalige Kristallisation nicht mehr.

Die aus dem Salz freigesetzte Base wurde in das Hydrochlorid überführt. Nach einmaligem Umkristallisieren aus Diisopropylether/Isopropanol 3:2 erhielt man 78,3 g (58 %) (R)-Verapamil-hydrochlorid vom Schmelzpunkt 129 bis 131°C und einem Drehwert $[\alpha]_D^{20} = +9{,}2°$ (Ethanol, c = 50 mg/ml). Dieser Wert änderte sich bei weiterem Umkristallisieren nicht mehr.

Beispiel 2

Das Beispiel 1 wurde wiederholt, jedoch wurde in a) (+)-O,O'-Dibenzoyl-D-weinsäure und in b) (-)-O,O'-Dibenzoyl-L-weinsäure verwendet. Das Ergebnis war dasselbe wie in Beispiel 1, jedoch erhielt man die Antipoden in umgekehrter Reihenfolge.

Beispiel 3

Man verfuhr wie in den Beispielen 1 und 2, verwendete aber an Stelle der optisch aktiven Formen der O,O'-Dibenzoylweinsäure die optisch aktiven Formen der O,O'-Di-4-toluoylweinsäure und erhielt so ebenfalls die (S)- bzw. (R)-Form des Verapamil-hydrochlorids.

Beispiel 4

Man verfuhr wie in den Beispielen 1a, b, 2 und 3, verwendete aber statt Methanol/Wasser = 2:1, Aceton/Wasser = 1:1 (doppeltes Volumen) und erhielt so ebenfalls die (R)- bzw. die (S)-Form des Verapamil-hydrochlorids.

Beispiel 5

250 g (0,55 Mol) racemisches Verapamil, 155 g (0,41 Mol) (-)-O,O'-Dibenzoyl-L-weinsäure-hydrat und 10 g (0,275 Mol) Chlorwasserstoff wurden unter Erwärmen in 2500 ml Methanol/Wasser = 1:1 gelöst. Das über Nacht ausgefallene Kristallisat wurde abgesaugt und wie in Beispiel 1 beschrieben umgelöst. Das Ergebnis war dasselbe wie in Beispiel 1. Das Verfahren wurde mit (+)-O,O'-Dibenzoyl-D-weinsäure wiederholt. Das Ergebnis war dasselbe wie in Beispiel 2. Mit den optisch aktiven Formen der O,O'-Ditoluylweinsäuren wurden ebenfalls die (S)- bzw. (R)-Form des Verapamil-Hydrochlorids erhalten.

## Patentansprüche

1. Verfahren zur Racemattrennung von Verapamil, dadurch gekennzeichnet, daß man die freie Base der Verbindung mit optisch aktiver Dibenzoylweinsäure oder Ditoluoylweinsäure im Molverhältnis 1:1 bis 1:2 in einem Methanol-Wasser-Gemisch im Verhältnis 1:1 bis 3:1 oder Aceton-Wasser-Gemisch im Verhältnis 0,5:1 bis 2:1 umsetzt, das so erhaltene Diastereomerengemisch durch Kristallisation trennt, danach die Diastereomeren in die freien Basen überführt und diese gegebenenfalls in ihre Salze mit physiologisch verträglichen Säuren überführt.

2. Verfahren gemäß Anspruch 1, dadurch gekennzeichnet, daß man bis zu 50 % der optisch aktiven Dibenzoylweinsäure oder Ditoluoylweinsäure durch Salzsäure ersetzt.

3. Verfahren gemäß Anspruch 1, dadurch gekennzeichnet, daß man das Verapamil mit optisch aktiver Dibenzoylweinsäure oder Ditoluoylweinsäure im Molverhältnis 1 : 0,75 in Gegenwart von 0,5 Mol Salzsäure umsetzt.

## Claims

1. A process for the resolution of racemic verapamil, which comprises reacting the free base of the compound with optically active dibenzoyltartaric acid or ditoluoyltartaric acid in the molar ratio from 1:1 to 1:2 in a methanol/water mixture in the ratio from 1:1 to 3:1 or acetone/water mixture in the ratio from 0.5:1 to 2:1, separating the mixture of diastereomers obtained in this way by crystallization, and then converting the distereomers into the free bases and these into their salts, if required, with physiologically tolerated acids.

2. A process as claimed in claim 1, wherein up to 50% of the optically active dibenzoyltartaric acid or ditoluoyltartaric acid is replaced by hydrochloric acid.

3. A process as claimed in claim 1, wherein the verapamil is reacted with optically active dibenzoyltartaric acid or ditoluoyltartaric acid in the molar ratio 1:0.75 in the presence of 0.5 mol of hydrochloric acid.

## Revendications

1. Procédé pour la séparation des racémiques du vérapamil, caractérisé par le fait qu'on fait réagir la base libre du composé avec de l'acide dibenzoyltartrique ou de l'acide ditoluoyltartrique optiquement actif dans le rapport molaire de 1:1 à 1:2 dans un mélange méthanol-eau dans le rapport de 1:1 à 3:1 ou dans un mélange acétone-eau dans le rapport de 0,5:1 à 2:1, on sépare par cristallisation le mélange de diastéréoisomères ainsi formé, on convertit les diastéréoisomères en bases libres et on transforme éventuellement celles-ci en leurs sels avec des acides physiologiquement acceptables.

2.  Procédé selon la revendication 1, caractérisé par le fait qu'on remplace par de l'acide chlorhydrique jusqu'à 50 % de l'acide dibenzoyltartrique ou de l'acide ditoluoyl-tartrique optiquement actif.

3.  Procédé selon la revendication 1, caractérisé par le fait qu'on fait réagir le vérapamil avec de l'acide dibenzoyltartrique ou de l'acide ditoluoyltartrique optiquement actif dans le rapport molaire de 1:0,75 en présence de 0,5 mol d'acide chlorhydrique.